# FASCICULE DE BREVET EUROPEEN

(11) **EP 0 544 571 B1**
(45) Date de publication et mention de la délivrance du brevet: **08.10.1997**
(21) Numéro de dépôt: 92403134.7
(22) Date de dépôt: 20.11.1992
(51) Int. Cl.: C10M 105/06, H01B 3/22, C07C 15/16

(54) **Composition à base de benzyltoluènes et de benzylxylènes**
Auf Benzyltoluol und Benzylxylol basierende Zusammensetzung
Composition based on benzyltoluene and benzylxylene

(30) Priorité: 26.11.1991 FR 9114585
(43) Date de publication de la demande: 02.06.1993
(73) Titulaire: ELF ATOCHEM S.A., 92800 Puteaux (FR)
(72) Inventeur: Commandeur, Raymond, F-38220 Vizylle (FR); Berger, Noelle, F-69130 Ecully (FR); Jay, Pierre, F-69370 Saint-Didier au Mont d'Or (FR)

(56) Documents cités:
- EP-A- 0 384 818
- EP-A- 0 444 989
- EP-A- 0 446 086

## Description

La présente invention concerne une composition a base de benzyltoluènes et de benzylxylenes et son application comme diélectrique.

On a déjà proposé dans le brevet EP 136 230 des compositions à base d'oligomères de benzyltoluène qui restent liquides à basse température sans présenter une viscosité élevée.

On a aussi proposé dans le brevet EP 299867 des compositions à base de (méthylbenzyl)xylène et d'homologues supérieurs.

Le brevet EP 446086 décrit des mélanges de benzylxylène, benzyltoluène, (méthylbenzyl)toluène et (méthylbenzyl)xylène et leur application comme diélectrique.

On a maintenant trouvé une nouvelle composition plus simple que la précédente et qui possède de meilleures propriétés diélectriques que les compositions décrites dans EP 136230 et EP 299865 en particulier la rigidité qui se mesure par la tension de claquage entre deux électrodes.

La composition selon l'invention est donc caractérisée en ce qu'elle comprend au moins un oligomère du benzyltoluène de formule A1. 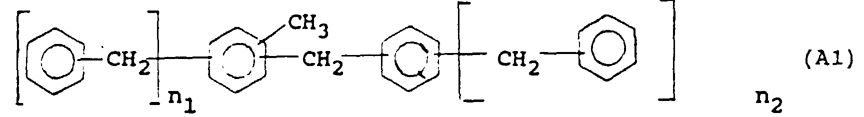 avec n₁ et n₂ = 0, 1 ou 2 sachant que n₁ + n₂ est inférieur ou égal à 3
et au moins un oligomère du benzylxylène de formule A2. 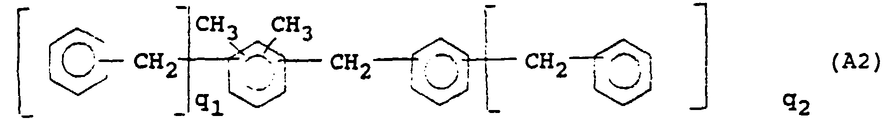 avec q₁ et q₂ = 0, 1 ou 2 sachant que q₁ + q₂ est inférieur ou égal à 3; à l'exclusion des (méthylbenzyl)toluènes et des (méthylbenzyl)xylènes.

On désigne par benzyltoluène le produit de formule A1 dans laquelle n₁ et n₂ valent 0. On désigne par benzylxylène le produit de formule A2 dans laquelle q₁ et q₂ valent 0.

La composition de l'invention est une huile isolante électrique utile dans les transformateurs de tension, les condensateurs ou les câbles électriques.

La composition de l'invention peut aussi comprendre des triphénylméthanes de formule B1 : 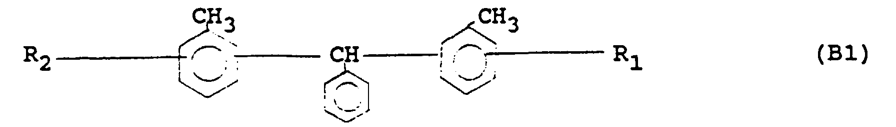 dans laquelle R₁ et R₂ identiques ou différents désignent l'hydrogène ou un méthyle.

On utilise avantageusement les compositions comprenant :
50 à 70 parties de benzyltoluène
15 à 25 parties de benzylxylène
10 à 20 parties d'un mélange quelconque de produits A1 et A2 dans lequel n₁ + n₂ = 1 et q₁ + q₂ = 1.

Ces 10 à 20 parties de produits triarylés peuvent comprendre aussi des triphénylméthanes de formule B1.

La demanderesse a trouvé que ces compositions ne sont pas cristallisées à -50°C.

Les compositions selon l'invention peuvent être préparées par mélange des produits A1, A2 et éventuellement B1.

A1 peut se préparer par condensation du chlorure de benzyle sur le toluène selon une réaction FRIEDEL et CRAFTS (FC). A2 peut se préparer par condensation du chlorure de benzyle sur le xylène selon une réaction FC. B1 peut se préparer par condensation du chlorure de benzylidène C₆H₅ - CHCl₂ sur le toluène, le xylène ou un mélange de toluène et de xylène.

Selon un forme avantageuse de l'invention on condense du chlorure de benzyle sur un mélange de toluène et de xylène, on prépare ainsi directement le mélange de A1 et A2 selon l'invention.

Si le chlorure de benzyle contient du chlorure de benzylidène alors il se forme aussi des triphénylméthanes de formule B1.

Cette condensation de FC est connue. On peut utiliser comme catalyseur un halogénure minéral ou un acide minéral. On utilise par exemple le chlorure d'aluminium, le chlorure ferrique ou l'acide sulfurique. Des condensations de FC ont été décrites dans les brevets EP 422986 et EP 435737 dont le contenu est incorporé dans la présente demande.

Selon une autre forme avantageuse de l'invention, on effectue une chloration radicalaire partielle du toluène pour obtenir un mélange de chlorure de benzyle et de toluène.

On peut éventuellement rectifier ce mélange pour éliminer le chlorure de benzylidène. On obtient donc au choix (i) un mélange de toluène et de chlorure de benzyle ou (ii) un mélange de toluène, de chlorure de benzyle et de chlorure de benzylidène.

Il suffit ensuite d'ajouter du xylène et d'effectuer une condensation de FC par exemple par addition de chlorure ferrique. Cette chloration radicalaire est connue en elle-même. Des chlorations similaires suivies de réaction FC ont été décrites dans les brevets EP 136230, EP 299867, EP 446086 dont le contenu est incorporé dans la présente demande.

Après la condensation de FC on élimine le catalyseur FC par lavage puis le toluène et le xylène en excès par une simple distillation. On peut aussi continuer la distillation pour récupérer la composition selon l'invention, il peut rester un fonds de produits lourds.

Il se peut que la composition de l'invention contienne un peu de chlore organique provenant de matières premières impures ayant des chlores sur les noyaux benzéniques. Il suffit d'effectuer un traitement avec un agent alcalin. On peut utiliser par exemple les procédés décrits dans les brevets EP 250748 et EP 306398 dont le contenu est incorporé dans la présente demande. Si on utilise le chlorure ferrique comme catalyseur FC, il n'est pas nécessaire de l'éliminer en fin de réaction de condensation FC. On élimine l'excès de toluène et de xylène par distillation, on continue la distillation pour récupérer la composition de l'invention, le fonds de distillation restant contient le chlorure de fer et les lourds éventuels. Ce procédé est décrit dans les brevets EP, 422986 et EP 435737 dont le contenu est aussi incorporé dans la présente demande.

Selon les différentes applications diélectriques il est avantageux de purifier la composition de l'invention à l'aide de terres décolorantes ou de zéolites puis d'ajouter des anti-oxydants ou des accepteurs d'acides. Tous ces conditionnements sont connus de l'homme de métier, on peut se reporter aux brevets EP 8251 et EP 136230.

On ne sortirait pas du cadre de l'invention si on ajoutait aux produits A1, A2 et éventuellement B1 d'autres huiles diélectriques ou des huiles minérales habituellement utilisées dans les transformateurs électriques.

### EXEMPLE 1

Dans un réacteur de 6 litres muni d'une agitation rotative, d'un réfrigérant ascendant dont la sortie est reliée à un barboteur à eau, on introduit par l'intermédiaire d'une ampoule de coulage 10 moles de chlorure de benzyle à 99 % de pureté dans un mélange de
34,8 moles de toluène
5,2 moles d'orthoxylène
contenant 2 g de FeCl₃. Le coulage s'effectue en 3 Heures à la température de 105°C. Le milieu réactionnel est maintenu encore 2 H à 100-110°C avec balayage par un courant d'azote (la quantité théorique d'HCl est recueillie dans le barboteur à eau).

Le toluène et l'orthoxylène non réagis sont distillés sur évaporateur rotatif sous un vide de 11.999 à 2000 Pa (90 à 15 mm de Hg), la température du bain de l'évaporateur variant entre 80 et 130°C.

Le résidu (1649 g) est placé dans un réacteur muni d'un agitateur. Il est porté à 150°C sous 2 133 Pa(16 mm Hg) de vide de manière à éliminer la quantité residuelle de toluène et d'orthoxylène (15,4 g).

Le résidu est ensuite traité par 36 g de méthylate de sodium, sous agitation et balayage à l'azote pendant 4 H 30 à la température de 293°C.

Le milieu ainsi traité est placé dans un ballon à distiller surmonté d'une colonne de 10 cm remplie par des gros anneaux de verre.

La distillation est réalisée sous un vide de 400 à 667 Pa (3 à 5 mm de Hg). On obtient une fraction de 961 g de composés à 2 cycles aromatiques avec une température de passage des vapeurs de 100 à 148°C. La distillation est poursuivie ce qui permet d'obtenir 338 g de fraction de composés à 3 cycles aromatiques dont les vapeurs passent entre 207 et 235°C. Les fractions de composés à 2 cycles aromatiques et à 3 cycles aromatiques (de type oligomères A1 et A2) sont mélangés dans une proportion pondérale de 84,8/15,2. Ce mélange à, d'après l'analyse chromatographique, une teneur de 65,2 % en benzyltoluènes de 19,2 % en benzylxylènes. Le reste étant des triaryles de formule A1 et A2. La teneur en chlore est de 2 ppm.

On appelle ce produit le BT/BX-A.

### EXEMPLE 2

Préparation identique à l'exemple 1 mais au lieu d'utiliser du chlorure de benzyle à 99 % de pureté on utilise 10 moles de chlorure brut. Ce chlorure de benzyle brut est obtenu par photochloration partielle de toluène conduisant au mélange suivant :
73,3 % de toluène
24,9 % de chlorure de benzyle
0,4 % de chlorure de benzylidène

L'excès de toluène est séparé par distillation. Le pied de distillation constitue le chlorure de benzyle brut ayant la composition suivante :
0,03 % de toluène
93,64 % de chlorure de benzyle
3,1 % de chlorure de benzylidène
le complément étant constitué par des oligomères de benzyltoluènes.

La condensation est effectuée avec un mélange de 34,8 moles de toluène et de 5,2 moles d'orthoxylène comme dans l'exemple 1. Nous obtenons après élaboration et distillation un mélange ayant la composition suivante en poids :
65,2 % de benzyltoluène
20,8 % de benzylxylène
13,9 % de composés à 3 cycles aromatiques.

La teneur en chlore est de 35 ppm.

Les composés à 3 cycles aromatiques sont un mélange d'oligomères de type A1 et A2 avec n₁ + n₂ = 1 q₁ + q₂ = 1 et d'oligomères B1. On appelle ce produit le BT/BX - B.

### EXEMPLE 3

Mode opératoire identique à l'exemple 2 mais en remplaçant l'orthoxylène par le paraxylène.

La composition du mélange final est la suivante
67,2 % de benzyltoluène
17,6 % de benzylxylène
14,9 % de composés à 3 cycles aromatiques.

Les composés à 3 cycles aromatiques sont du même type que ceux de l'exemple 2.

La teneur en chlore est de 142 ppm. On appelle ce produit le BT/BX-C.

### EXEMPLE 4 (tests diélectriques)

On a mesuré la rigidité et on l'a comparée à d'autres huiles diélectriques.

Le BT 06 est un mélange d'oligomères du benzytoluène selon EP 136230 constitué de :
80 parties de benzyltoluène, le produit A1 tel que n₁ + n₂ = O.
15 parties de produit A1 tel que n₁ + n₂ = 1
5 parties d'un mélange :
   a/ de produit A1 avec n₁ + n₂ = 2
   b/ de produit B1 dans lequel R₁ et R₂ sont des hydrogène

Le XXO 5 est un mélange d'oligomères du (méthylbenzyl)xylène selon EP 443899 constitué de :
85 parties de 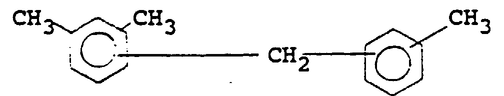
12 parties de produit 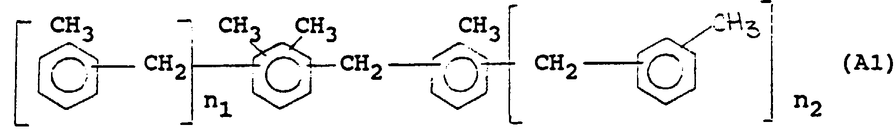 tel que n₁ + n₂ = 1
3 parties de

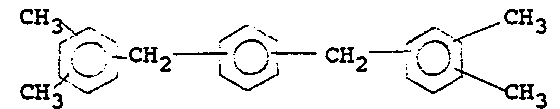

Les mesures de tension de claquages ont été effectuées dans les conditions suivantes :
- Electrodes : une pointe de phonographe (⌀ ≈ 50 µm) et un disque ROGOWSKI
- Rampe de 3000 Volts/seconde
- Distance entre électrodes = 5 mm
- Température ambiante.

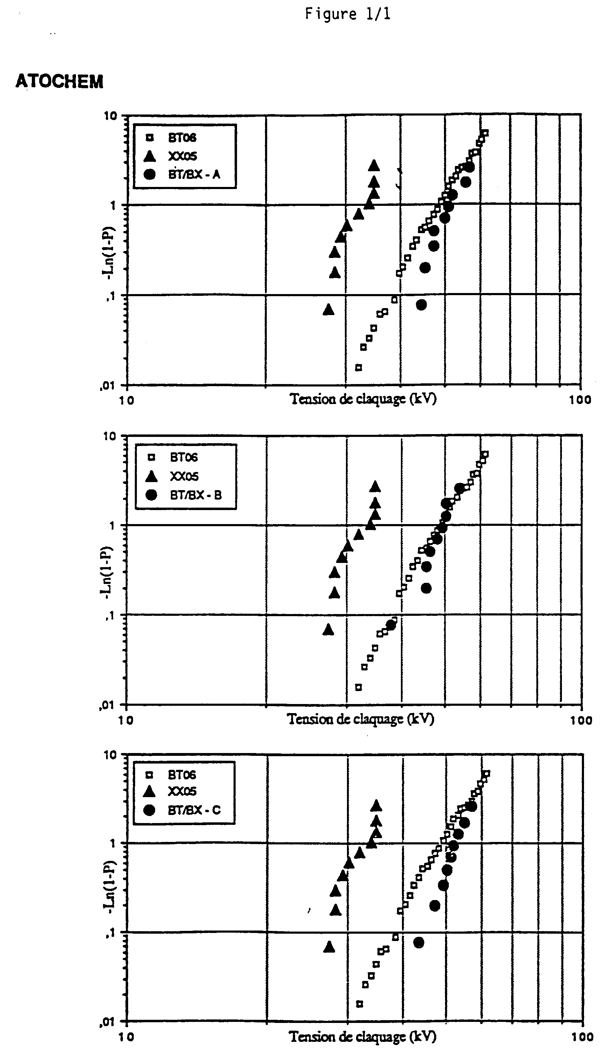

Sur la figure 1 ci-après, les mesures ont été reportées sur des diagrammes de Weibull qui sont couramment utilisés pour représenter la distribution statistique de la rigidité diélectrique des matériaux.

Ces diagrammes montrent que la rigidité des mélanges BT/BX est nettement supérieure à celle du XX05, et légèrement meilleure que celle du BT06. C'est également ce que l'on constate à partir des moyennes :

| LIQUIDE | XX05 | BT06 | BT/BX-A | BT/BX-B | BT/BX-C |
|---|---|---|---|---|---|
| Tension de claquage moyenne (kV) | 31,4 | 46,6 | 50,0 | 47,4 | 50,9 |

Sur la Figure 1-Ln (1-P) représente le logarithme népérien de (1-P), P est la probabilité cumulée.

## Revendications

1. Composition caractérisée en ce qu'elle comprend au moins un oligomère du benzyltoluène de formule A1 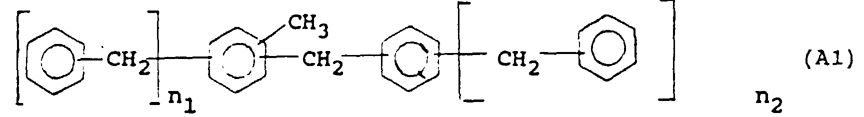 avec n₁ et n₂ = 0, 1 ou 2 sachant que n₁ + n₂ est inférieur ou égal à 3,
au moins un oligomère du benzylxylène de formule A2 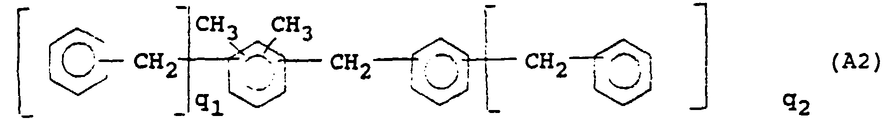 avec q₁ et q₂ = 0, 1 ou 2 sachant que q₁ + q₂ est inférieur ou égal à 3 et en ce qu'elle ne comprend pas des (méthylbenzyl)-toluènes et des (methylbenzyl)xylènes.

2. Composition selon la revendication 1 caractérisée en ce qu'elle comprend :
50 à 70 parties de benzyltoluène
15 à 25 parties de benzylxylène
10 à 20 parties d'un mélange quelconque de produits A1 et A2 dans lequel n₁ + n₂ = 1 et q₁ + q₂ = 1.

3. Composition selon la revendication 1 ou 2 caractérisée en ce qu'elle comprend aussi des triphénylméthanes de formule B1 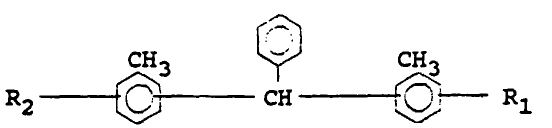 dans laquelle R₁ et R₂ identiques ou différents désignent l'hydrogène ou un méthyle.

## Patentansprüche

1. Zusammensetzung, dadurch gekennzeichnet, daß sie mindestens ein Benzyltoluol-Oligomer der Formel **A1** 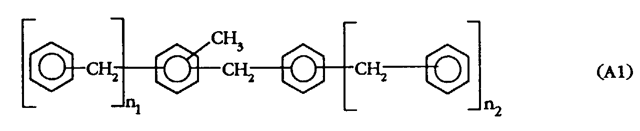 mit n₁ und n₂ = 0, 1 oder 2, wobei n₁ + n₂ kleiner als oder gleich 3 ist, und mindestens ein Benzylxylol-Oligomer der Formel **A2** 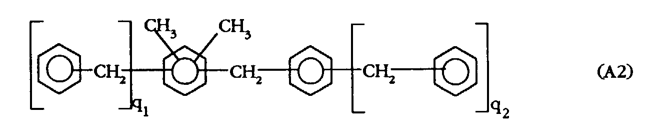 mit q₁ und q₂= 0, 1 oder 2, wobei q₁ + q₂ kleiner als oder gleich 3 ist, umfaßt und daß sie keine (Methylbenzyl)toluole und (Menthylbenzyl)xylole umfaßt.

2. Zusammensetzung nach Anspruch 1, dadurch gekennzeichnet, daß sie umfaßt:
50 bis 70 Teile Benzyltoluol
15 bis 25 Teile Benzylxylol
10 bis 20 Teile einer beliebigen Mischung aus den Produkten **A1** und **A2**, wobei n₁ + n₂ = 1 und q₁ + q₂ = 1 sind.

3. Zusammensetzung nach Anspruch 2, dadurch gekennzeichnet, daß sie außerdem Triphenylmethane der Formel **B1** 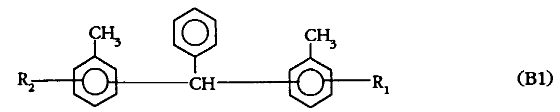 umfaßt, in der R₁ und R₂, identisch oder verschieden, Wasserstoff oder einen Methylrest bezeichnen.

## Claims

1. Composition, characterized in that it comprises at least one oligomer of the benzyltoluene of formula A1 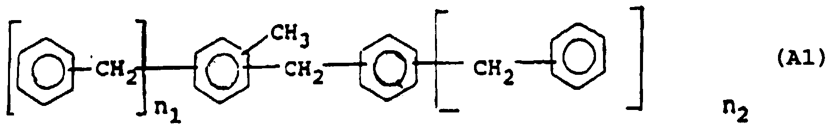 with n₁ and n₂ = 0, 1 or 2 knowing that n₁ + n₂ is less than or equal to 3,
at least one oligomer of the benzylxylene of formula A2 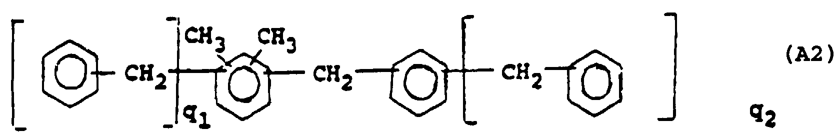 with q₁ and q₂ = 0, 1 or 2 knowing that q₁ + q₂ is less than or equal to 3, and in that it does not comprise (methylbenzyl)toluenes and (methylbenzyl)xylenes.

2. Composition according to Claim 1, characterized in that it comprises:
50 to 70 parts of benzyltoluene
15 to 25 parts of benzylxylene
10 to 20 parts of any mixture of products A1 and A2 in which n₁ + n₂ = 1 and q₁ + q₂ = 1.

3. Composition according to Claim 1 or 2, characterized in that it also comprises triphenylmethanes of formula B1 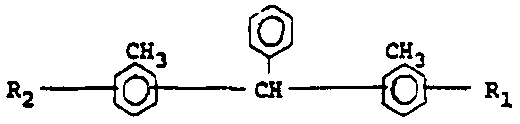 in which R₁ and R₂, which are identical or different, denote hydrogen or a methyl.
